# EUROPEAN PATENT APPLICATION

(11) **EP 2 239 000 A2**
(43) Date of publication of application: **13.10.2010**
(21) Application number: 10002254.0
(22) Date of filing: 04.03.2010
(51) Int. Cl.: A61M 5/42, A61B 5/15, A61B 5/00

(54) **System for measuring components in a living body, kit for a micropore forming device, and marking member**

(30) Priority: 13.03.2009 JP 2009060369
(71) Applicant: Sysmex Corporation, Kobe-shi, Hyogo 651-0073 (JP)
(72) Inventor: Asakura, Yoshihiro, Kobe-shi Hyogo 651-0073 (JP)
(74) Representative: HOFFMANN EITLE

(57) **Abstract**

A system for measuring analytes in a living body comprising: a micropore forming device for forming a micropore on the skin, a guide member mounted on one end of the micropore forming device, a marking member for indicating a position on the skin on which micropores are formed, a collecting member for collecting tissue fluid from the skin via micropores, and a measuring apparatus for measuring analytes in a living body contained in the tissue fluid collected by the collecting member, is disclosed.

A kit for a micropore forming device and a marking member are also disclosed.

## Description

### FIELD OF THE INVENTION

The present invention relates to a system for measuring components in a living body, a kit for a micropore forming device, and a marking member. Specifically, the present invention relates to a system for measuring components in a living body that collects tissue fluid from the skin of a living body and measures components contained in collected tissue fluid, a kit for a micropore forming device used to collect tissue fluid from the skin of a living body, and a marking member.

### BACKGROUND

There are known conventional methods for collecting tissue fluid from the skin of a living body.

U.S. Patent Application Publication No. 20030113827 discloses a method for collecting tissue fluid by forming micropore pathways in the skin through collision of particles on the skin, and subsequently adhering an dressing for collecting the tissue fluid in the processing region in which the micropore pathways have been formed on the skin. A problem arises with this tissue fluid collecting method in as much as is difficult to position the dressing on the processing region because it is considered to be difficult to visually verify the processing region in which the micropore pathways have been formed. U.S. Patent Application Publication No. 20030113827 does not make any mention of the positioning of the dressing on the processing region.

There are known conventional techniques capable of easily positioning a collection member on a processing region.

U.S. Patent Application Publication No. 20070233011 discloses a blood glucose level analyzer that includes a main body having a detachable extraction cartridge for collecting tissue fluid, a receiver for positioning a piercing device for forming micropores in the skin, and a belt for mounting the receiver on the wrist of a user. The receiver has an opening for exposing the skin, and is configured so that the processing region of the skin in which micropores have been formed by the piercing device is exposed through this opening. The main body is mounted on the receiver via a hinge so as to be rotatable, and is configured so that the mounted extraction cartridge is disposed over the processing region of the skin. In this way the extraction cartridge can be easily positioned relative to the processing region. Tissue fluid can then be extracted by the extraction cartridge while the receiver is mounted on the wrist via the belt.

### SUMMARY OF THE INVENTION

The scope of the invention is defined solely by the appended claims, and is not affected to any degree by the statements within this summary.

A first aspect of the present invention is a system for measuring components in a living body comprising: a micropore forming device for forming micropores by moving a microneedle chip toward a skin; a guide member comprising: a mounting part being mounted on one end of the micropore forming device; and a positioning part for positioning the micropore forming device on the skin, wherein the guide member is provided with a channel to permit passage of the microneedle chip when the microneedle chip is moved toward the skin; a marking member, for indicating a position on the skin on which micropores are formed, comprising: a first adhesive surface adhered to the positioning part; and a second adhesive surface, on the back surface of the first adhesive surface, adherable to the skin by a greater adhesion force than the adhesion force of the first adhesive surface to the positioning part; a collecting member, for collecting tissue fluid from the skin, placed at a position indicated by the marking member on the skin; and a measuring apparatus for measuring components in a living body contained in the tissue fluid collected by the collecting member. Herewith, a position of a micropore formed on a skin is indicated by the marking member. Furthermore, the collecting member is easily placed on the position on which the micropores are formed by placing the collecting member on the position indicated by the marking member. Furthermore, the marking member is able to be adhered to an arm of user without using attachment device like a belt, by the second adhesive surface provided on the marking member. Accordingly, burden of user can be reduced.

Preferably, the marking member comprises: a first marking member provided on the positioning part; and a second marking member provided at a position, opposite the first marking member with the opening of the channel, on the positioning part, wherein the first marking member and the second marking member respectively comprise the first adhesive surface and the second adhesive surface.

Preferably, the marking member has a configuration capable of surrounding the opening of the channel of the guide member.

Preferably, the collecting member comprises: a collecting part for collecting tissue fluid; and a holding part, for holding the collecting part, that is adherable to the skin, wherein the marking member has a predetermined configuration, and the holding part has a configuration correspond to the configuration of the marking member. Herewith, the marking member is easily placed on micropores.

Preferably, the collecting part is a gel; and the holding part is a film. Herewith, the gel is easily placed on the skin.

Preferably, the holding part is water-proof, and is large enough to cover the collecting part placed on the skin. Herewith drying of the collecting member placed on the skin is prevented.

Preferably, the marking member is configured as a sheet. Herewith, the marking member is able to be deformed according to motion of user. Accordingly, burden of user is reduced.

A second aspect of the present invention is a kit for a micropore forming device for use in a micropore forming device for forming micropores by moving a microneedle chip toward a skin, comprising: a guide member comprising: a mounting part being mounted on one end of the micropore forming device; and a positioning part for positioning the micropore forming device on the skin, wherein the guide member is provided with a channel to permit passage of the microneedle chip when the microneedle chip is moved toward the skin; a marking member, for indicating a position on the skin on which micropores are formed, comprising: a first adhesive surface adhered to the positioning part; and a second adhesive surface, on the back surface of the first adhesive surface, adherable to the skin by a greater adhesion force than the adhesion force of the first adhesive surface to the positioning part. Herewith, a position of a micropore formed on a skin is indicated by the marking member. Furthermore, the collecting member is easily placed on the position on which the micropores are formed by placing the collecting member on the position indicated by the marking member. Furthermore, the marking member is able to be adhered to an arm of user without using attachment device like a belt, by the second adhesive surface provided on the marking member. Accordingly, burden of user can be reduced.

Preferably, the marking member comprises: a first marking member provided on the positioning part; and a second marking member provided at a position, opposite the first marking member with the opening of the channel, on the positioning part, wherein the first marking member and the second marking member respectively comprise the first adhesive surface and the second adhesive surface.

Preferably, the marking member has a configuration capable of surrounding the opening of the channel of the guide member.

Preferably, the kit further comprises: a collecting member for collecting tissue fluid from the skin by being placed at a position on the skin on which micropores have been formed.

Preferably, the collecting member comprises: a collecting part for collecting tissue fluid; and a holding part, for holding the collecting part, that is adherable to the skin, wherein the marking member has a predetermined configuration, and the holding part has a configuration correspond to the configuration of the marking member. Herewith, the marking member is easily placed on micropores.

Preferably, the collecting part is a gel; and the holding part is a film. Herewith, the gel is easily placed on the skin.

Preferably, the holding part is water-proof, and is large enough to cover the collecting part placed on the skin. Herewith drying of the collecting member placed on the skin is prevented.

Preferably, the marking member is configured as a sheet. Herewith, the marking member is able to be deformed according to motion of user. Accordingly, burden of user is reduced.

A third aspect of the present invention is a marking member, for indicating a position on a skin on which micropores have been formed, configured to adhere to a guide member, wherein the guide member comprises a mounting part being mounted on one end of the micropore forming device for forming micropores by moving a microneedle chip toward the skin and a positioning part for positioning the micropore forming device on the skin, wherein the guide member is provided with a channel to permit passage of the microneedle chip when the microneedle chip is moved toward the skin, the marking member comprising: a first adhesive surface adhered on the positioning part of the guide member; and a second adhesive surface, on the back surface of the first adhesive surface, adherable to the skin, wherein the marking member has a configuration capable of surrounding the opening of the channel.

### BRIEF DESCRIPTION OF THE DRAWINGS

FIG. 1 is a perspective view showing the general structure of the system for measuring components in a living body of a first embodiment of the present invention;
FIG. 2 is a cross section view showing the structure of the piercing device of the first embodiment of the system for measuring the components in a living body pf FIG. 1;
FIG. 3 is a plan view showing the structure of the measuring apparatus of the first embodiment of the system for measuring the components in a living body of FIG. 1;
FIG. 4 is a block diagram showing the structure of the measuring apparatus of the first embodiment of the system for measuring the components in a living body of FIG. 1;
FIG. 5 is a perspective view showing the bottom surface of the skin guide of the system for measuring the components in a living body of the first embodiment shown in FIG. 1;
FIG. 6 is a plan view showing the positioning tape of the first embodiment of the system for measuring the components in a living body of FIG. 1;
FIG. 7 is a cross sectional view showing the unused positioning tape of the first embodiment of the system for measuring the components in a living body of FIG. 1;
FIG. 8 is a cross sectional view illustrating the structure of the positioning tape of the first embodiment of the system for measuring the components in a living body of FIG. 1;
FIG. 9 is a plan view showing the collection member of the first embodiment of the system for measuring the components in a living body of FIG. 1;
FIG. 10 is a cross sectional view showing the collection member of the first embodiment of the system for measuring the components in a living body pf FIG. 1;
FIG. 11 is a cross sectional view illustrating the method of using the first embodiment of the system for measuring the components in a living body of FIG. 1;
FIG. 12 is a perspective view illustrating the method of using the first embodiment of the system for measuring the components in a living body of FIG. 1;
FIG. 13 is a cross sectional view illustrating the method of using the first embodiment of the system for measuring the components in a living body of FIG. 1;
FIG. 14 is a plan view illustrating the method of using the first embodiment of the system for measuring the components in a living body of FIG. 1;
FIG. 15 is a plan view illustrating the method of using the first embodiment of the system for measuring the components in a living body of FIG. 1;
FIG. 16 is a perspective view showing the structure of the positioning tape of a second embodiment of the system for measuring the components in a living body of the present invention;
FIG. 17 is a plan view showing the positioning tape of the second embodiment of the system for measuring the components in a living body of FIG. 16;
FIG. 18 is a plan view showing the collection member of the second embodiment of the system for measuring the components in a living body of FIG. 16;
FIG. 19 is a plan view illustrating the method of adhering the collection member of the second embodiment of the system for measuring the components in a living body of FIG. 16;
FIG. 20 is a cross sectional view showing the unused positioning tape of a third embodiment of the system for measuring the components in a living body of the present invention;
FIG. 21 is a cross sectional view illustrating the method of adhering the positioning tape to the skin guide of the third embodiment of the system for measuring the components in a living body of FIG. 20;
FIG. 22 is a plan view illustrating modifications of the first and third embodiments of the present invention; and
FIG. 23 is a plan view illustrating modifications of the first through third embodiments of the present invention.

### DETAILED DESCRIPTION OF THE PREFERRED EMBODIMENTS

Hereinafter, embodiments of the invention will be described in detail with reference to the accompanying drawings.

### [First Embodiment]

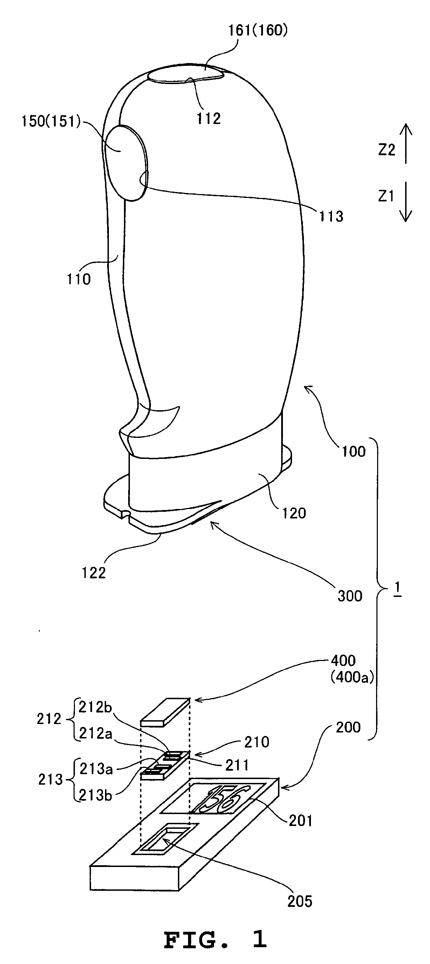

The structure of a system for measuring components in a living body 1 of the first embodiment is described hereinafter with reference to FIGS. 1 through 10.

The system for measuring the components in a living body 1 of the first embodiment of the present invention is configured by a piercing device 100, measuring apparatus 200, positioning tape 300, and collecting member 400 that includes a collecting part 400a, as shown in FIG. 1.

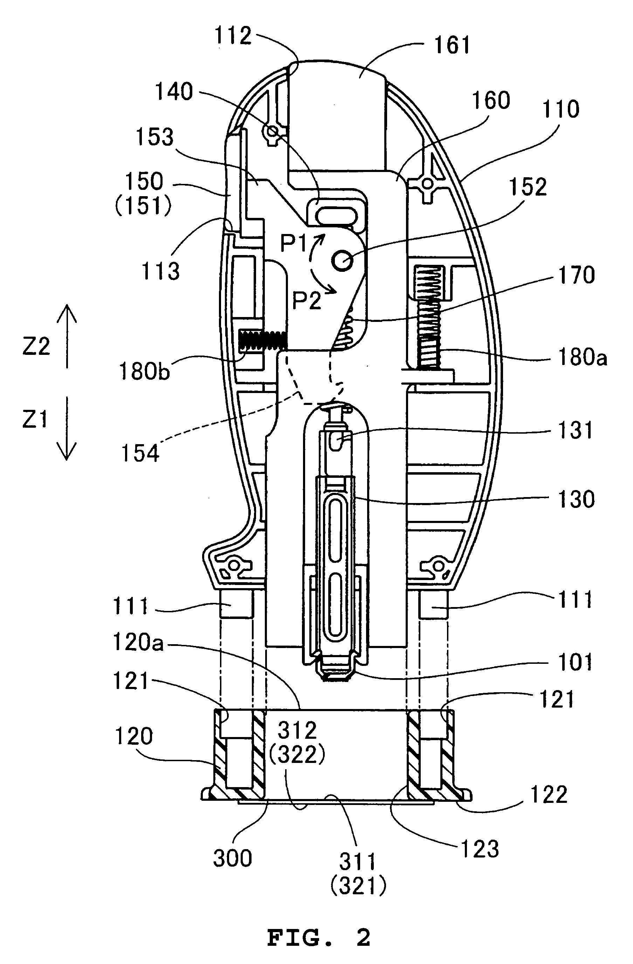

The piercing device 100 is a device for forming body fluid extraction holes (micropores) in the skin 500 by mounting a sterilized microneedle chip 101 (refer to FIG. 2), and bringing the microneedle chip 101 into contact with the skin 500 of a living body. The body fluid (tissue fluid) that exudes from the micropores formed in the skin 500 by the piercing device 100 is then collected by the collecting part 400a, and the glucose level is obtained by the measuring apparatus 200 which measures the collected tissue fluid.

The piercing device 100 pierces the corneum of the skin 500 and reaches the border between the intraepidermis and the corium so as to form a plurality of micropores to a depth that does not reach the deep portion of corium. As shown in FIGS. 1 and 2, the piercing device 100 is provided with a housing 110, skin guide 120, chuck array 130 (refer to FIG. 2), spring stopper 140 (refer to FIG. 2), release mechanism 150, ejector 160, main spring 170 (refer to FIG. 2), and springs 180a and 180b (refer to FIG. 2). Note that except for the springs (main spring 170, springs 180a and 180b), the six members (housing 110, skin guide 120, chuck array 130, spring stopper 140, release mechanism 150, and ejector 160) are respectively formed of resin.

As shown in FIG. 2, the housing 110 is configured so as to internally house the chuck array 130, spring stopper 140, release mechanism 150, ejector 160, main spring 170, and springs 180a and 180b. A mounting part 111 is provided at the bottom of the housing 110 for mounting the skin guide 120. An opening 112 is provided at the top of the housing 110 to expose a button 161 of the ejector 160. An opening 113 is provided at the side of the housing 110 to expose a button 151 of the release mechanism 150.

The skin guide 120 is disposed on a mount 120a for mounting on the piercing device 100, as shown in FIG. 2. The skin guide 120 includes an insertion hole 121 for the insertion of the mounting part 111 of the housing 110, a bottom 122 that is disposed on the skin 500 of a arm 600 of a user (patient) when performing the piercing by the piercing device 100 and that functions as a positioning part for positioning the piercing device 100 on the skin 500, and a through hole 123 that is configured so that the skin guide 120 can pass through from the mount 120a to the bottom 122. The through hole 123 has a diameter sufficiently large to allow the passage of both the chuck array 130 provided within the housing 110, and the microneedle chip 101 (described later) for forming the micropores in the skin 500 when mounted on the chuck array 130. Thus, when piercing, the microneedle chip 101 mounted on the chuck array 130 passes through the through hole 123 of the skin guide 120 in conjunction with the movement of the chuck array 130 in the Z1 direction and comes into contact with the skin 500.

In the first embodiment, the skin guide 120 is configured to hold the positioning tape 300 on the surface of the bottom 122. Specifically, the bottom 122 is flat, and the positioning tape 300 and the bottom 122 are adhered via the adhesion of the device-side adhesion surface 311 (321) of the positioning tape 300 (refer to FIG. 2). A silicon coating is also provided on the bottom 122. Thus, the adhesion of the bottom 122 and the device-side adhesion surface 311 (321) of the positioning tape 300 is less than the adhesion of the skin-side adhesion surface 312 (322) (refer to FIG. 2) of the skin 500 and the positioning tape 300.

The chuck array 130 is configured to be vertically movable (Z1 and Z2 directions) as shown in FIG. 2. The bottom end of the chuck array 130 is also configured to hold the microneedle chip 101. The chuck array 130 is pressed downward (Z1 direction) by the main spring 170 as will be described later. The chuck array 130 has, near the top end, a connector 131 for engaging a stationary part 154 (described later) of the release mechanism 150. The chuck array 130 is configured so that the connector 131 engages the stationary part 154 of the release mechanism 150 by moving upward (Z2 direction) against the force exerted by the main spring 170.

The spring stopper 140 is provided to support the main spring 170 exerting a downward force (Z1 direction) on the chuck array 130.

The release mechanism 150 has a button 151, a body 153 configured to be rotatable around a support shaft 152, and an anchor 154 that integrally rotates with the body 153. The anchor 154 has the function of fixedly anchoring the chuck array 130 moving upward (Z2 direction) against the downward (Z1 direction) force exerted by the main spring 170. Specifically, the anchor 154 is configured to anchor the chuck array 130 that has received the force exerted by the main spring 170 by engaging the connector 131 of the chuck array 130. The release mechanism 150 is further configured to release the engagement of the connector and the anchor 154 when the button 151 is pressed and the body 153 is rotated in the P1 direction. The chuck array 130 is thereby moved downward (Z1 direction) via the force exerted by the main spring 170.

The ejector 160 has the function of ejecting a chip receiver (not shown in the drawing), which is used when mounting the microneedle chip 101 on the chuck array 130, through the through hole 123 of the skin guide 120. Specifically, the ejector 160 is configured to push the chip receiver, which has been inserted in the through hole 123 of the skin guide 120, downward (Z1 direction) when mounting the microneedle chip 101.

The main spring 170 is provided to exert a downward force (Z1 direction) on the chuck array 130.

The spring 180a has the function of exerting a downward (Z1 direction) force on the ejector 160 that has been lifted upward (Z2 direction). The spring 180b is provided to rotate, in the P2 direction, the body 153 of the release mechanism 150 that has rotated in the P1 direction pivoting on the support shaft 152. Two springs (not shown in the drawings) are provided within the housing 110 to repel, upward (Z2 direction), the chuck array 130, which has been moved downward (Z1 direction) by the force exerted by the main spring 170. Thus, the chuck array 130 that has been moved downward (Z1 direction) passes a predetermined position and can be prevented form moving further downward (Z1 direction), and as a result the microneedles of the microneedle chip 101 are prevented from deeply piercing the arm 600.

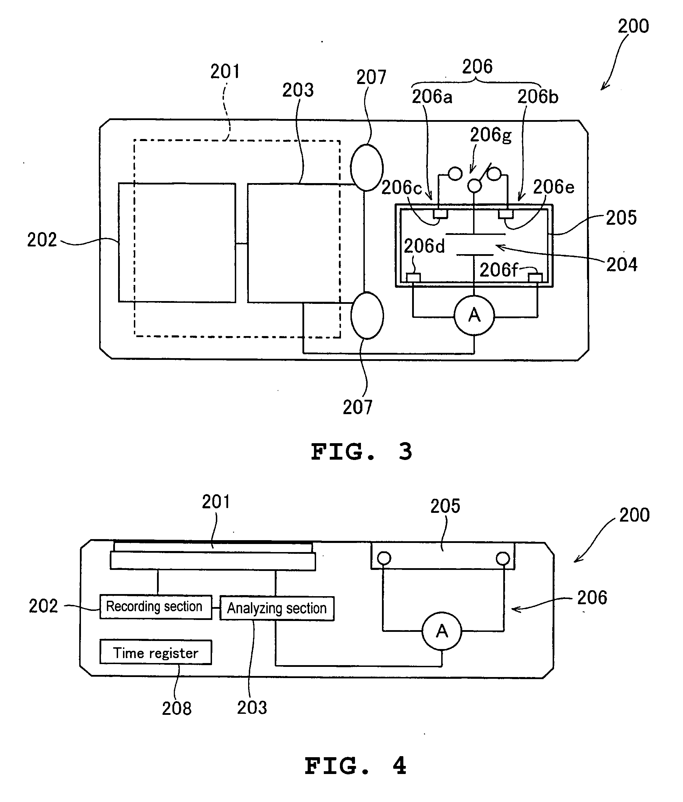

As shown in FIGS. 1, 3, and 4, the measuring apparatus 200 is provided with a display 201, recording section 202 (refer to FIGS. 3 and 4), analyzing section (refer to FIGS. 3 and 4), power source 204 (refer to FIG. 3), installation 205 for installing a sensor chip 210 (refer to FIG. 1) and collecting part 400a (refer to FIG. 1), electric circuit 206 connected to the sensor chip 210 installed in the installation 205 (refer to FIGS. 3 and 4), operation button 207 for user operation of the measuring apparatus 200 (refer to FIG. 3), and time register 208 (refer to FIG. 4).

The display 201 has the function of displaying the measurement results of the analyzing section 203 and the data recorded by the recording section 202. The recording section 202 is provided to store data of the past. The analyzing section 203 has the function of calculating the glucose level and electrolyte (NaCl) concentration based on the output value of the electric circuit 206. The installation 205 has a concave configuration for installing the sensor chip 210 and the collecting part 400a. The electric circuit 206 includes a glucose measuring circuit 206a and an electrolyte measuring circuit 206b, as shown in FIG. 3. The glucose measuring circuit 206a includes terminals 206c and 206d which are exposed within the installation 205, and the electrolyte measuring circuit 206b includes terminals 206e and 206f which are exposed within the installation 205. The electric circuit 206 also includes a switch 206g for swotching between the glucose measuring circuit 206a and the electrolyte measuring circuit 206b. A user (patient) can switch between the glucose measuring circuit 206a and the electrolyte measuring circuit 206b by operating the switch 206g via the operation of an operation button 207. The operation button 207 is provided for operations such as setting the time register 208, changing the display of the display 201, and switching by the switch 206g. The time register 208 has the function of alerting the user (patient) that the extraction time has ended in order to end the extraction in a predetermined time after starting glucose extraction.

As shown in FIG. 1, the sensor chip 210 includes a plastic board 211, a pair of glucose measurement electrodes 212 provided on the top surface of the board 211, and a pair of electrolyte measurement electrodes 213 provided on the top surface of the board 211. The glucose measurement electrodes 212 are configured by an acting electrode 212a formed of a GOD (GOD: glucose oxidase) enzyme membrane on a platinum electrode and an opposed electrode 212b formed of a platinum electrode; the electrolyte measurement electrodes 213 are configured by an acting electrode 213a formed of silver/silver chloride and an opposed electrode 213b formed of silver/silver chloride. When the sensor chip 210 is installed in the installation 205 of the measuring apparatus 200, the acting electrode 212a and the opposed electrode 212b of the glucose measurement electrodes 212 are respectively in contact with the terminals 206c and 206d of the glucose measuring circuit 206a. Similarly, when the sensor chip 210 is installed in the installation 205 of the measuring apparatus 200, the acting electrode 213a and the opposed electrode 213b of the electrolyte measurement electrodes 213 are respectively in contact with the terminals 206e and 206f of the electrolyte measuring circuit 206b.

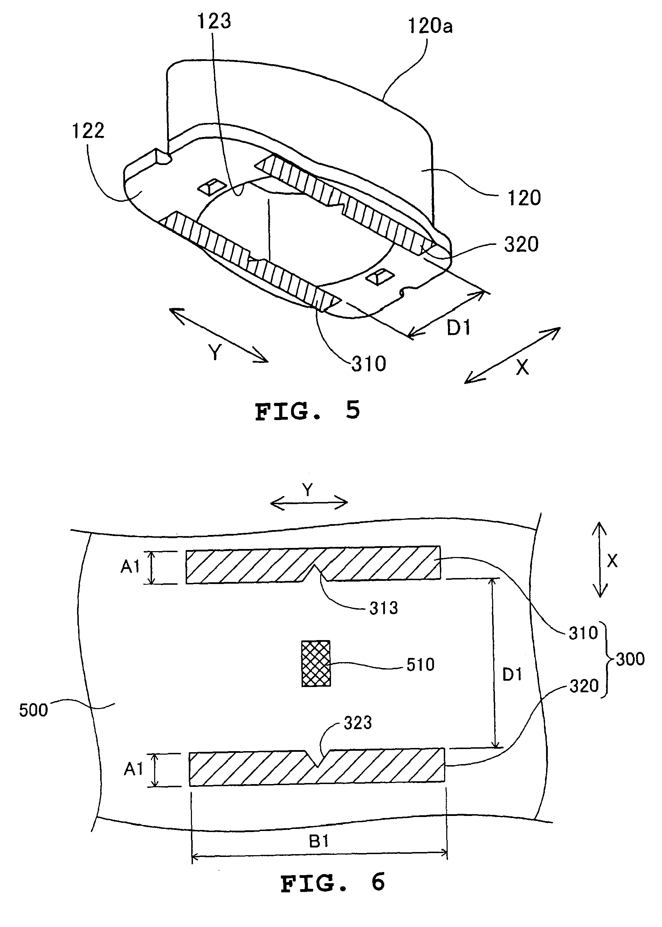

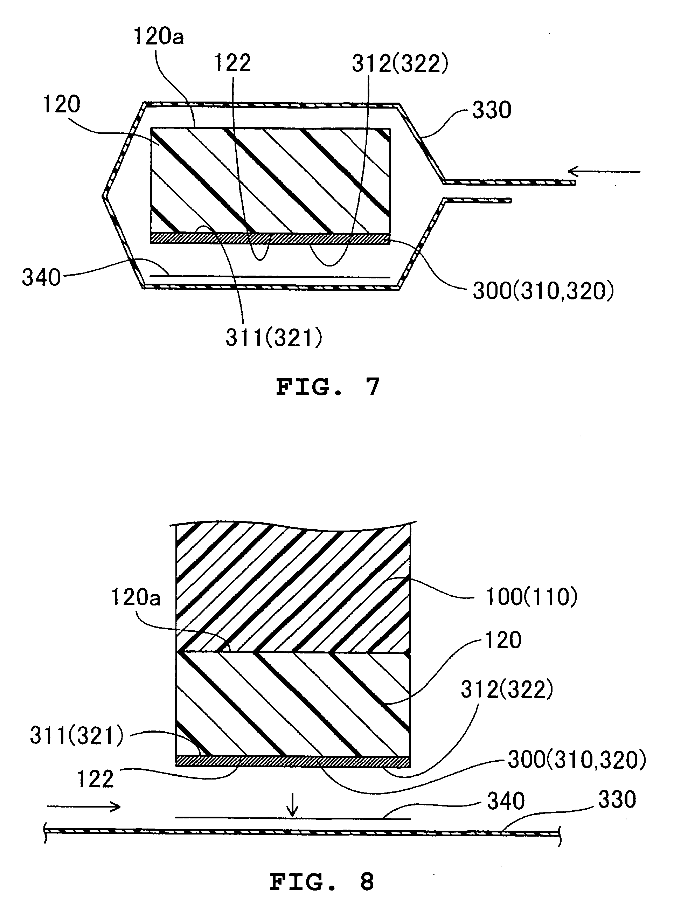

In the first embodiment, the positioning tape 300 is composed of a sheet-like pair of double-sided tape pieces 310 and 320, as shown in FIGS. 5 and 6. Configuring the positioning tape 300 as a sheet renders the positioning tape 300 readily deformable in conjunction with the movement of the skin 500 of the user (patient). The positioning tape 300 may also be visually adhered to the skin 500. The pair of two-sided tape pieces 310 and 320 are affixed to the top surface of the bottom 122 of the skin guide 120, as shown in FIGS. 5 and 7. Specifically, the pair of two-sided tape pieces 310 and 320 respectively have a device-side adhesion surface 311 and 321, and a skin-side adhesion surface 312 and 322, and the device-side adhesion surface 311 and 321 are adhered to the bottom 122 of the skin guide 120. The device-side adhesion surface 311 (321) and the skin-side adhesion surface 312 (322) are respectively sticky. Since the silicon coating is provided on the bottom 122 of the skin guide 120 as previously mentioned, the device-side adhesion surface 311 (321) and the bottom 122 of the skin guide 120 can be adhered with a relatively small adhesive force. The skin-side adhesion surface 312 (322) is configured so as to be adhered to the skin 500 of the user (patient), and the adhesive force between the skin-side adhesion surface 312 (322) and the skin 500 is greater than the adhesive force between the device-side adhesion surface 311 (321) and the bottom 122 of the skin guide 120.

As shown in FIG. 5, the pair of two-sided tapes 310 and 320 are adhered to the bottom 122 of the skin guide 120 with a mutual spacing of distance D1 (approximately 30 mm) in the X direction. The pair of two-sided tapes 310 and 320 also are adhered to the bottom 122 of the skin guide 120 so as to move the microneedles of the microneedle chip 101 to the approximate center of the surrounding region. In other words, the two-sides tapes 310 and 320 are disposed so as to circumscribe the opening of the through hole 123 of the skin guide on the surface of the bottom 122. That is, the two-sided tapes 310 and 320 are supported by the skin guide 120 so as to indicate the position of the through hole 123 of the skin guide 120. Thus, a processing region (region in which micropores are formed) 510 (refer to FIG. 6) is formed by the piercing device 100 in the approximate center of the region circumscribed by the two-side positioning tape 300 when the positioning tape 300 is adhered to the skin 500.

The pair of two-sided tape pieces 310 and 320 are rectangular and respectively have a length A1 in the X direction of approximately 2 mm and a length B1 in the Y direction of approximately 32 mm, as shown in FIG. 6. The pair of two-sided tape pieces 310 and 320 have triangular notches 313 and 323 formed on the mutually facing sides. The two notches 313 and 323 are disposed in the approximate center of each mutually facing side.

When unused, the positioning tape 300 is packaged in a film 330 as a kit for a micrpore forming device together with the skin guide 120 to which the positioning tape 300 is adhered, as shown in FIG. 7. Specifically, the skin guide 120 and the positioning tape 300 are packaged in the film 330 in a state wherein the device-side adhesion surface 311 (321) of the positioning tape 300 is adhered to the bottom 122 of the skin guide 120 and the entirety of the surface of the skin-side adhesion surface 312 (322) is covered by a peel-off paper 340.

A silicon coating is provided on the entire surface of the side of the peel-off paper 340 in contact with the skin-side adhesion surface 312 (322) in order to make easy to be peeled off. Thus, there is relatively slight adhesive force between the peel-off paper 340 and the skin-side adhesion surface 312 (322) of the positioning tape 300. The backside surface of the peel-off paper 340 that contacts the positioning tape is fixedly attached to the inside surface of the film 330. As shown in FIG. 8, the skin-side adhesion surface 312 (322) of the positioning tape is thus exposed when the peel-off paper 340 is moved with the film 330 as the film 330 is peeled away when the skin guide 120 and positioning tape 300 are removed from within the film 330. Note that, although a silicon coating is provided on the peel-off paper 340 and the bottom 122 of the skin guide 120, respectively, only the peel-off paper 340 is easily released since the peel-off paper 340 is readily deformable compared to the skin guide 120. Therefore, the sticky skin-side adhesion surface 312 (322) is exposed by peeling the film 330 when the device-side adhesion surface 311 (321) of the positioning tape 300 is adhered to the bottom 122 of the skin guide 120.

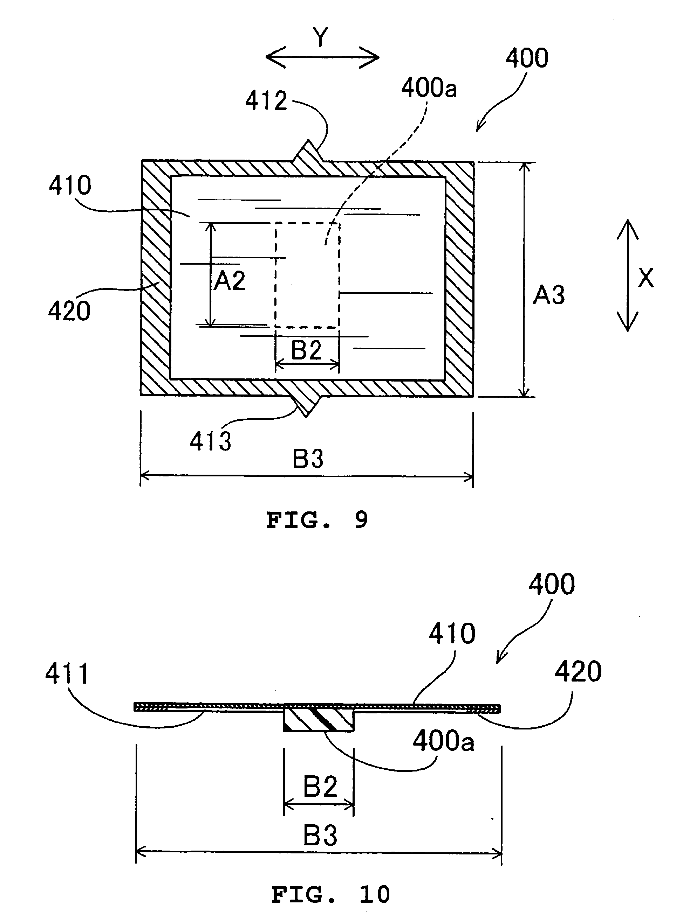

The collecting member 400 includes the collecting part 400a and a holding part 410. The collecting part 400a is a gel member that collects tissue fluid exuded from the micropores formed by the piercing device 100. As shown in FIG. 9, the collecting part 400a is approximately rectangular with a length A2 in the X direction of approximately 12 mm and a length B2 in the Y direction of approximately 7 mm in the planar view. The collecting part 400a also has a flat configuration slightly larger than the processing region (region in which micrpores are formed) created by the piercing device 100. As shown in FIGS. 9 and 10, the entirety of the top surface (backside of the surface adhered to the skin) of the collecting part 400a is adhered to the approximate center of the holding part 410.

The holding part 410 is film-like (thin film) and has an adhesion surface 411 which can adhere to the skin 500. The holding part 410 holds the collecting part 400a on the surface of the adhesion surface 411. The holding part 410 adheres the collecting part 400a to the skin 500 while covering the collecting part 400a by adhering the adhesion surface 411 to the skin 500. The holding part 410 has a size sufficient to cover the collecting part 400a and is approximately rectangular with a length A3 in the X direction of approximately 28 mm and a length B3 in the Y direction of approximately 28 mm. That is, the length B3 in the Y direction of the holding part 410 (approximately 28 mm) is slightly smaller than the distance D1 (approximately 30 mm) in the X direction between the pair of two-sided tape pieces 310 and 320. The holding part 410 functions as a positioning part of the collecting member (described later), and has triangular projections 412 and 413 at the approximate center on the respective pair of outer side edges. These two projections 412 and 413 are respectively formed with shapes corresponding to the two notches 313 and 323 of the positioning tape 300. The holding part 410 is moisture proof. Since the holding part 410 is moisture proof, drying out of the gel member collecting part 400a can be prevented.

A frame-like or plate-like reinforcing member 420 configured by, for example, PET resin is disposed on the outer edge of the holding part 410. As shown in FIGS. 9 and 10, the reinforcing member 420 is adhered on the surface of the adhesion surface 411 of the holding part 410 along the outer edge of the holding part 410. The reinforcing member 420 is provided to reinforce the film-like (thin film) holding part 410. Specifically, the reinforcing member 420 is configured to spread the film-like (thin film) holding part 410 that can be easily twisted into a flat shape. The reinforcing member 420 is sticky on the surface on the opposite side from the side on which the holding part 410 is adhered, so as to be adherable to the skin 500.

The method of using the system for measuring the components in a living body 1 is described below with reference to FIGS. 1, 6 through 8, and 10 through 15.

As shown in FIG. 7, the skin guide 120, on which the positioning tape 300 is adhered, packaged in the film 330 are first removed from the film 330. Specifically, the film 330 is peeled from the top surface of skin guide on the opposite side from the bottom 122. Thereafter, the skin guide 120 is adhered to the piercing device 100 by inserting the mounting part 111 of the housing 110 of the piercing device 100 into the insertion hole 121 of the skin guide 120, as shown in FIG. 8. The film 330 is then peeled from the bottom 122 side of the skin guide 120 (side with the positioning tape 300). The peel-off paper 340 fixed to the film 330 is thus peeled off from the skin-side adhesion surface 312 (322) of the positioning tape 300 thereby exposing the sticky skin-side adhesion surface 312 (322).

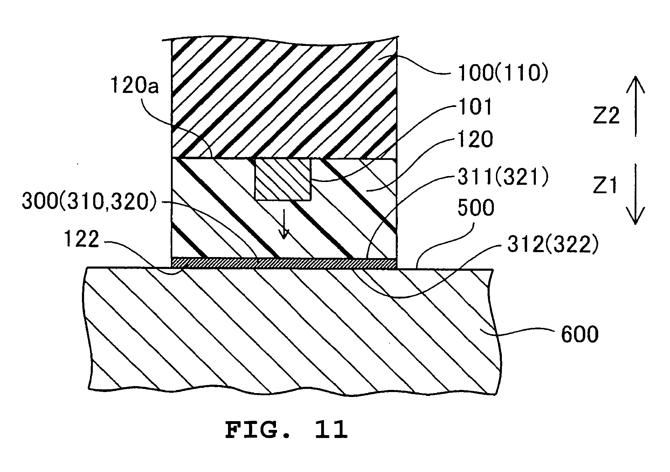

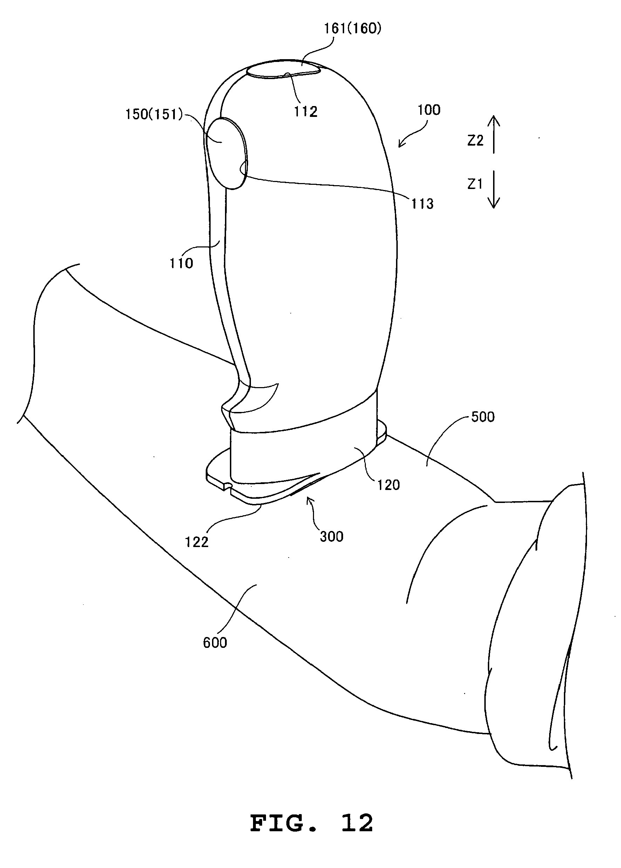

As shown in FIG. 11, the microneedle chip 101 is then inserted from the through hole 123 (refer to FIG. 2) of the skin guide 120, and the microneedle chip 101 is adhered to the chuck array 130 (refer to FIG. 2) of the piercing device 100. As shown in FIGS. 11 and 12, the bottom 122 of the skin guide 120 is then arranged on the skin 500 of the arm 600 of the user (patient) to adhere the skin-side adhesion surface 312 (322) of the positioning tape 300 on the skin 500 of the arm 600 of the user (patient). In this state, the microneedle chip 101 passes beyond the positioning tape 300 and protrudes to the skin 500 side (Z1 direction side) by pressing the button 151 of the piercing device 100. Micropores are thus formed in the skin 500 of the arm 600 of the user (patient).

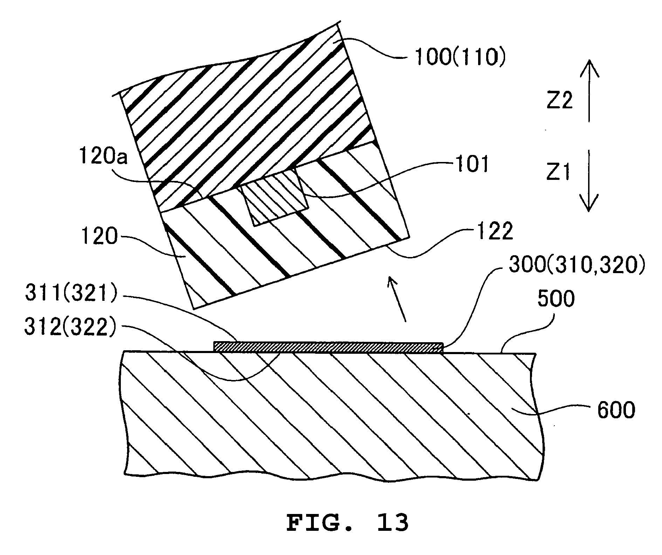

As shown in FIG. 13, the piercing device 100 and the skin guide 120 are thereafter separated from the skin 500. Since the adhesive force between the skin 500 and the skin-side adhesion surface 312 (322) is greater than the adhesive force between the device-side adhesive surface 311 (321) and the bottom 122 of the skin guide 120, the positioning tape 300 is peeled from the skin guide 120 while still adhered to the skin 500. As shown in FIGS. 6 and 13, the positioning tape 300 thus remains on the skin 500. That is, the positioning tape 300 is adhered to the skin 500 during the processing performed by the piercing device 100, and the positioning tape 300 is detached form the skin guide 120 while remaining adhered to the skin 500 after the processing performed by the piercing device 100. The pair of two-sided tape pieces 310 and 320 of the positioning tape 300 are adhered on the surface of the skin 500 so that the processing region (region in which micropores are formed) 510 of the piercing device 100 is in the approximate center of the circumscribed region.

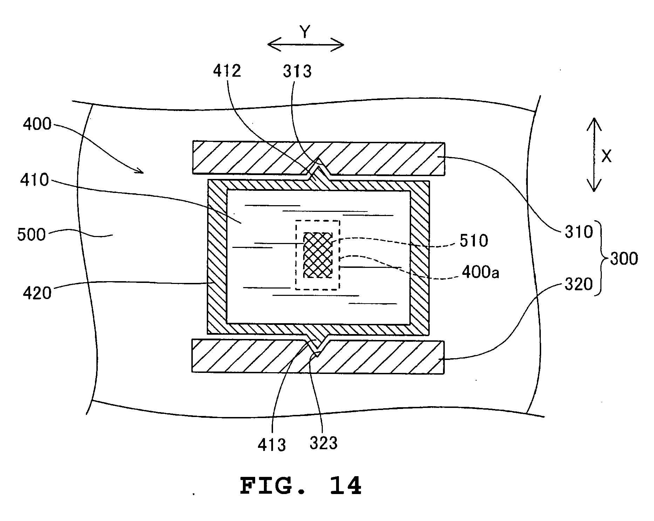

As shown in FIG. 14, the holding part 410 holding the collecting part 400a is adhered to the skin 500 so that the adhesive surface 411 (refer to FIG. 10) abuts the skin 500. Specifically, the holding part 410 is disposed on the skin 500 so that the projections 412 and 413 of the holding part 410 correspond with the notches 313 and 323 of the positioning tape 300. Thus, the holding part 410 is adhered to the skin 500 so that the collecting part 400a held in the approximate center of the holding part 410 covers the processing region (region in which micropores are formed) 510 of the piercing device 100. That is, the positioning tape 300 and the holding part 410 are used as the positioning member of the collecting member 400 (collecting part 400a and holding part 410) in order to adhere the collecting part 400a on the processing region (region in which micropores are formed) 510.

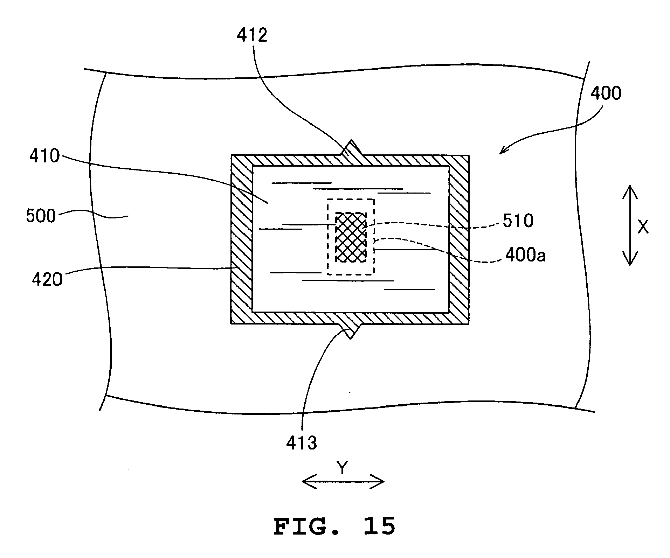

After the collecting part 400a has been adhered to the processing region (region in which micropores are formed) 510 by adhering the holding part 410 on the skin 500, the positioning tape 300 (310, 320) is peeled from the skin 500 as shown in FIG. 15. Thus, since only the collecting part 400a and holding part 410 remain on the skin 500 while the tissue fluid is collected by the collecting part 400a, the burden on the user (patient) is reduced compared to when the positioning tape 300 (310, 320) remained on the skin 500.

After the tissue fluid has been fully collected by the collecting part 400a (after approximately one hour from the adhesion of the collecting part 400a to the processing region 510), the collecting member 400 with completed tissue fluid collection is installed on the top surface of the sensor chip 210 installed in the installation 205 of the measuring apparatus 200. A first circuit is thus formed by the gel member (collecting part) 400a and the glucose measuring electrode 212 of the sensor chip 210 and the glucose measuring circuit 206a of the measuring apparatus 200. At the same time, a second circuit is formed by the gel member (collecting part) 400a and the electrolyte measuring electrode 213 of the sensor chip 210 and the electrolyte measuring circuit 206b of the measuring apparatus 200.

When measuring the glucose level of the extracted glucose, the switch 206g is switched to the glucose measuring circuit 206a by the operation button 207, and a start measurement instruction is issued. Thus, a fixed voltage of a predetermined value is applied to the first circuit, and the electrical current value detected by the ammeter is input to the analyzing section 203. The detected electrical current value is proportional to the concentration of the glucose contained in the collecting part 400a. Relationships based on the current values and glucose concentrations are pre-stored in the analyzing section 203, so that the glucose concentration within the collecting part 400a can be calculated by the analyzing section 203 based on the stored relationships and the input electrical current value.

In the first embodiment, the electrolyte concentration contained in the collecting part 400a can also be measured by the measuring apparatus 200. In this case, the user (patient) operates the operation button 207 to switch the switch 206g to the electrolyte measuring circuit 206b, and a start measurement instruction is issued. Thus, a fixed voltage of a predetermined value is applied to the second circuit, the original electrolyte electromotive force generated in the second circuit is detected by a voltmeter, and the detected voltage value is input to the analyzing section 203. The detected voltage value is proportional to the electrolyte concentration contained in the collecting part 400a. Relationships based on the voltage values and electrolyte concentrations are pre-stored in the analyzing section 203, so that the electrolyte concentration within the collecting part 400a can be calculated by the analyzing section 203 based on the stored relationships and the input voltage value.

Thereafter, the analyzing section 203 corrects the glucose concentration based on the calculated electrolyte concentration, and stores the corrected glucose concentration in the recording section 202. The measurement result stored in the recording section 202 is then displayed on the display 201.

The significance of the correction of the glucose level based on the electrolyte concentration is described below. Although glucose is accumulated in the collecting part 400a while contained in the tissue fluid, the glucose must be quantified coupled with the condition of the micropore formation since the quantity of tissue fluid exuded outside the body fluctuates depending on the state of the micropore formation. On the other hand, electrolytes are essentially thought to be present at a constant concentration in a living body, and therefore the concentration of the electrolytes accumulated in the collecting part 400a after a predetermined extraction time has elapsed increases if the micropores have enlarged and decreases if the micropores have reduced in size. That is, the electrolyte concentration can be considered to reflect the state of micropore formation. Thus, a more accurate glucose concentration reflecting the state of micropore formation can be determined by correcting the glucose concentration based on the electrolyte concentration contained in the collecting part 400a.

By providing a skin guide 120 for forming the through hole 123 through the bottom 122 from the mount 120a to allow the passage of the microneedle chip 101 when the microneedle chip 101 is moved toward the skin 500 by the piercing device 100 and including therein a bottom 122 that functions as a positioning part for positioning the piercing device 100 on the skin 500, and providing a positioning tape 300 having a device-side adhesion surface 311 (321) adhered to the bottom 122 and a skin-side adhesion surface 312 (322), disposed on the back side of the device-side adhesion surface 311 (321), so as to be adherable to the skin 500 by an adhesive force greater than the adhesive force between the bottom 122 and the device-side adhesion surface 311 (321), and which is held by the skin guide 120 so that the position of the through hole 123 is over the bottom 122, the first embodiment described above can form micropores in the skin 500 at a position corresponding to the through hole 123 of the skin guide 120 on the skin 500 when the microneedle chip 101 of the piercing device 100 is moved toward the skin 500 to pass through the through hole 123 formed in the skin guide 120 while the skin-side adhesion surface 312 (322) of the positioning tape 300 retained by the bottom 122 of the skin guide 120 is adhered to the skin 500 after the skin guide 120 has been installed in the piercing device 100. the positioning tape 300 remains on the skin 500 when the skin guide 120 adhered to the piercing device 100 is separated from the skin 100 because the adhesive force of the skin-side adhesion surface 312 (322) of the positioning tape 300 relative to the skin 500 is greater than the adhesive force of the of the device-side adhesion surface 311 (321) relative to the bottom 122 of the skin guide 120. In this case, the positioning tape 300, which is retained on the skin guide 120 to indicate the position of the through hole 123 on the bottom 122, is disposed on the skin 500 so as to indicate the position corresponding to the through hole 123 of the skin guide 120 on the skin 500. Thus, the position of the micropores formed at the position corresponding to the through hole 123 of the skin guide 120 on the skin 500 is well defined by the positioning tape 300. The collecting member 400 can also be easily positioned relative to the position of the micropores defined by the positioning tape 300 when providing the collecting member 400 to collect tissue fluid from the skin 500 by adhering the collecting member 400 at the position on the skin 500 indicated by the positioning tape 300. The positioning tape 300 can be adhered to the arm 600 without using a mounting device such as a belt or the like since the positioning tape 300 can be easily adhered to the skin 500 by providing the positioning tape 300 with a skin-side adhesion surface 312 (322) that is adherable to the skin 500. In this way the burden on the user is reduced. The burden on the user is accordingly further reduced because the collecting member 400 can be easily position relative to the micropores (processing region) formed in the skin 500.

In the first embodiment, the position of the micropores formed in the skin 500 at a position corresponding to the through hole 123 of the skin guide 120 is well defined by the positioning tape 300 by providing, as the positioning tape 300, a two-sided tape piece 310 disposed on the bottom 122 of the skin guide 120 and a two-sided tape piece 320 disposed at a position on the opposite side of the two-sided tape piece 310 so as to interpose therebetween the opening of the through hole 123 on the bottom 122 and positioning the two-sided tape pieces 310 and 320 so that the through hole 123 of the skin guide 120 on the skin 500 is interposed between the two-sided tap pieces 310 and 320 when the positioning tape 300 remains on the skin 500.

In the first embodiment, the collecting part 400a can be easily adhered to the region in which the micropores are formed by simply positioning the triangular projection 412 (413) of the holding part 410 relative to the notch 313 (323) of the positioning tape 300 by providing collecting member 400 with a collecting part 400a for collecting tissue fluid and a holding part 410 for holding the collecting part 400a and adhering the collecting part 400a to the skin 500 and configuring the collecting member 400 so that the collecting part 400a adheres to the region in which the micropores are formed by forming the triangular projection 412 (413) on the holding part 410 and positioning the projection 412 (413) relative to the notches 313 (323) of the positioning tape 300 adhered to the skin 500, then adhering to the holding part 410.

In the first embodiment, the positioning tape 300 (310, 320) can remain on the skin 500 after the processing performed by the piercing device 100 by configuring the positioning tape 300 (310, 320) so that the adhesive force between the skin-side adhesion surface 312 (322) of the positioning tape 300 (310, 320) and the skin 500 is greater than the adhesive force between the device-side adhesion surface 311 (321) of the positioning tape 300 (310, 320) and the bottom 122 of the skin guide 120, adhering the skin-side adhesion surface 312 (322) to the skin with the device-side adhesion surface 311 (321) is adhered to the bottom 122 of the skin guide 120, and thereafter separating the skin guide 120 from the skin 500.

In the first embodiment, the collecting part 400a held by the holding part 410 can be adhered easily to the processing region 510 because the holding part 410 can be easily positioned by providing the triangular notch 313 (323) in the positioning tape 300 (310, 320), and arranging the holding part 410 so that the projection 412 (413) of the holding part 410 that holds the collecting part 400a of the collecting member 400 corresponds to the triangular notch 313 (323).

In the first embodiment, the burden on the user (patient) is reduced when adhering the positioning tape 300 on the skin 500 because the region in which the positioning tape 300 is adhered is smaller when using the positioning tape 300 configured by a pair of two-sided tape pieces 310 and 320 than when a frame-like positioning tape is used.

### [Second Embodiment]

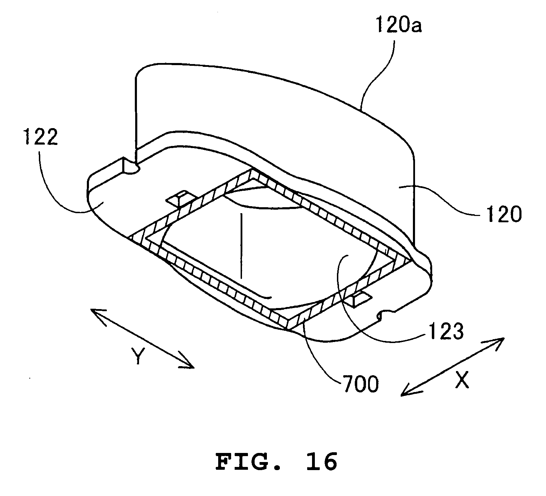

The second embodiment is described below referring to FIGS. 16 through 19. The second embodiment differs from the first embodiment in that a positioning tape 700 configured by a single frame-like two-sided tape piece is used.

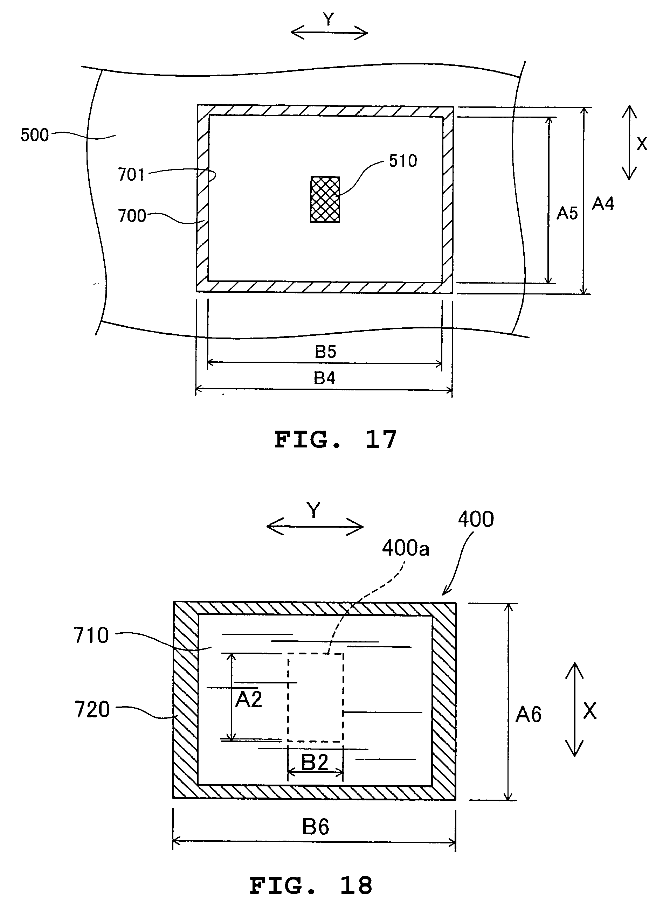

In the second embodiment, the positioning tape 700 is configured by a sheet-like single two-sided tape piece as shown in FIGS. 16 and 17. The positioning tape 700 is approximately rectangular with an external shape having a length A4 in the X direction of approximately 32 mm and a length B4 in the Y direction of approximately 32 mm, as shown in FIG. 17. The positioning tape 700 also has an approximately rectangular empty space 701 that has a length A5 in the X direction of approximately 30 mm and a length B5 in the Y direction of approximately 30 mm. That is, the positioning tape 700 is frame-like in shape and the frame is approximately 1 mm in width. The frame-like positioning tape 700 is adhered to the bottom 122 of the skin guide 120 so that the microneedles of the microneedle chip 101 move to the approximate center of the circumscribed region, as shown in FIG. 16. In other words, the positioning tape 700 has a shape which approximately circumscribes the opening through hole 123 on the surface of the bottom 122 of the skin guide 120. Thus, a processing region (region in which micropores are formed) 510 is formed by the piercing device 100 in the approximate center of the region circumscribed by the positioning tape 700 when the positioning tape 700 has been adhered to the skin 500, as shown in FIG. 17.

The collecting member 400 includes the collecting part 400a and a holding part 710. The holding part 710 is configured to hold the collecting part 400a in the approximate center in the planar view as shown in FIG. 16. The holding part 710 is a film (thin film). The holding part 710 has a size sufficient to cover the collecting part 400a and is approximately rectangular with a length A6 in the X direction of approximately 28 mm and a length B6 in the Y direction of approximately 28 mm. That is, the approximately rectangular holding part 710 (length A6 in the X direction (approximately 28 mm) and length B6 in the Y direction (approximately 28 mm)) is slightly smaller than the approximately rectangular empty space 701 (length A5 in the X direction (approximately 30 mm) and length B5 in the Y direction (approximately 30 mm)) of the positioning tape 700. A reinforcing member 720 is disposed on the outer edge of the holding part 710. The reinforcing member 720 is provided along the exterior edge of the holding part 710 as shown in FIG. 18.

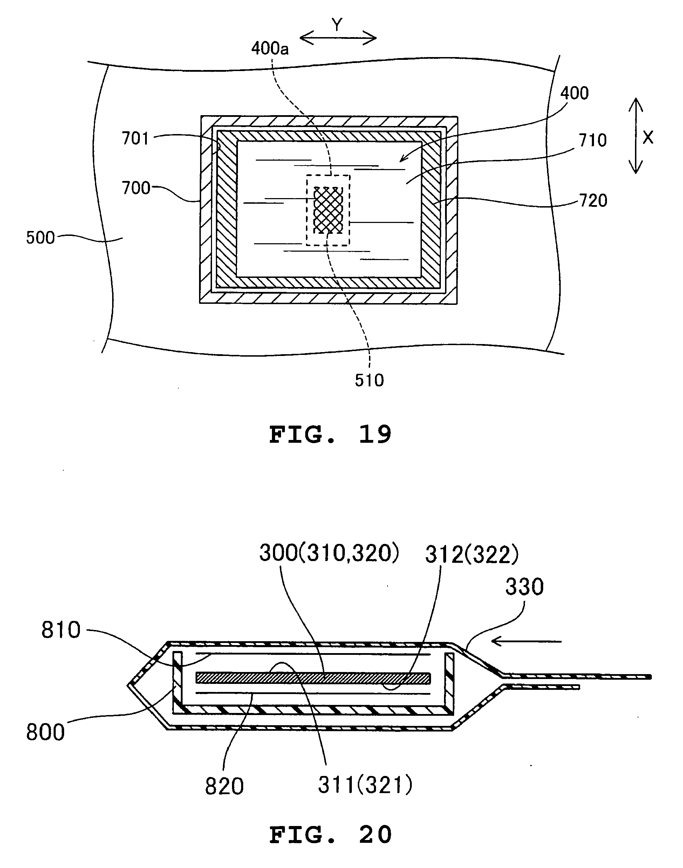

The method of adhering the collecting member 400 to the skin 500 in the second embodiment is described below referring to FIGS. 17 and 19.

After the positioning tape 700 has been adhered to the skin 500 as shown in FIG. 17, the holding part 710 which holds the collecting part 400a is adhered to the skin 500 as shown in FIG. 19. Specifically, the holding part 710 is disposed so that the four outside edges of the holding part 710 are along the four inside edges of the frame-like positioning tape 700.That is, the holding part 710 is arranged within the empty space 701 of the positioning tape 700 which has a flat configuration slightly larger than the external shape of the holding part 710. Thus, the holding part 710 is adhered to the skin 500 so that the collecting part 400a held in the approximate center of the holding part 710 covers the processing region (region in which micropores are formed) 510 of the piercing device 100.

The positioning tape 700 is peeled from the skin 500 after the collecting part 400a has been adhered to the processing region (region in which micrpores are formed) 510 by adhering the holding part 710 to the skin 500. Thus, since only the collecting part 400a and holding part 710 remain on the skin 500 while the tissue fluid is collected by the collecting part 400a, the burden on the user (patient) is reduced compared to when the positioning tape 700 remained on the skin 500.

Note that in other aspects the structure of the second embodiment is identical to that of the first embodiment.

In the second embodiment described above, the positioning tape 700 clearly defines the position of the micropores formed in the skin 500 at a position corresponding with the through hole 123 of the skin guide 120 by forming the positioning tape 700 so as to circumscribe the opening of the through hole 123 on the bottom 122 of the skin guide 120 and arranging the positioning tape 70 so as to approximately circumscribe the position on the skin 500 corresponding to the through hole 123 of the skin guide 120 when the positioning tape 700 remains on the skin 700.

In the second embodiment, the burden of the user (patient) is further reduced compared to the first embodiment by using the positioning tape 700 configured by one frame-like two-sided tape piece and peeling off the entire positioning tape 700 via a single peeling operation when the positioning tape 700 is separated from the skin 500. after the holding part 710 has been adhered to the skin 500.

Note that in other aspects the effect of the second embodiment is identical to that of the first embodiment.

### [Third Embodiment]

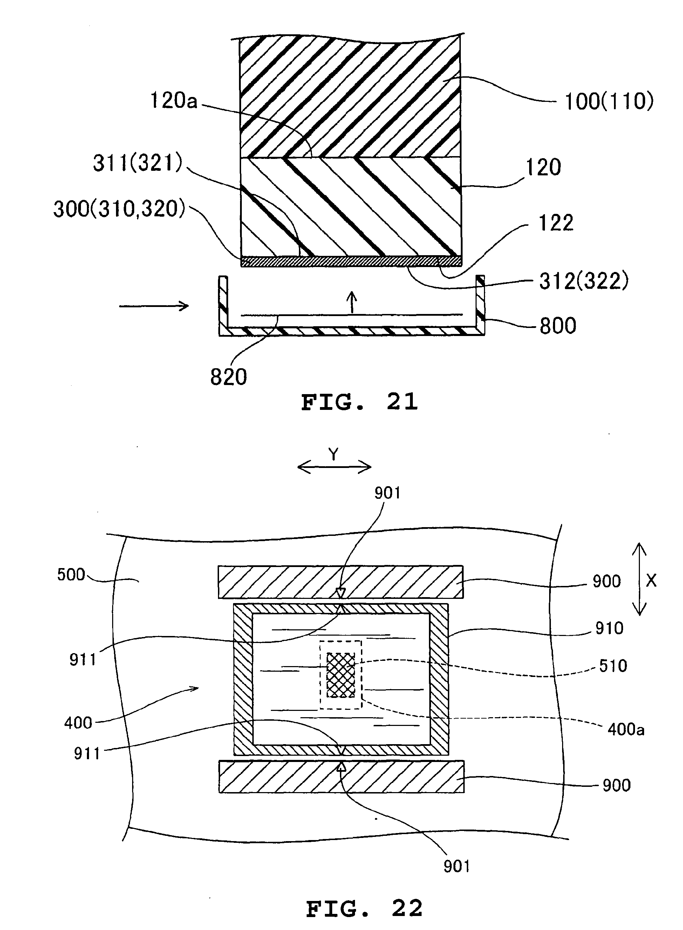

The third embodiment is described below referring to FIGS. 20 and 21. The third embodiment differs from the first embodiment in that the positioning tape 300 is configured to be packaged in the film 330 with the unused positioning tape 300 separated from the skin guide 120.

In the third embodiment, the positioning tape 300 is packaged in a film 330 together with a concave holder 800 for holding the unused positioning tape 300 as shown in FIG. 20. Specifically, the positioning tape 300 is packaged in the film 330 with the entirety of the surface of the device-side adhesion surface 311 (321) covered by a peel-off paper 810, and the entirety of the surface of the skin-side adhesion surface covered by a peel-off paper 820.

The holder 800 is configured to hold the positioning tape 300 at a predetermined position within the concavity. The holder 800 is also configured be fitted into the skin guide 120 from the bottom side (bottom 122 side) of the skin guide 120 so as to cover the bottom 122 of the skin guide 120 installed in the piercing device 100 as will be described later.

A silicon coating is provided on the entirety of the surface of the peel-off paper 810 on the side abutting the device-side adhesion surface 311 (321). A silicon coating is also provided on the entirety of the surface of the peel-off paper 820 on the side abutting the skin-side adhesion surface 312 (322). Thus, the peel-off papers 810 and 820 and the positioning tape 300 are adhered by an adhesive force allowing easy separation. The surface of the peel-off paper 810 on the opposite side of the side abutting the device-side adhesion surface 311 (321) is fixedly attached to the inside surface of the film 330. The surface of the peel-off paper 820 on the opposite side of the side abutting the skin-side adhesion surface 312 (322) is fixedly attached to the inside surface of the holder 800.

The method of adhering the positioning tape 300 to the bottom 122 of the skin guide 120 in the third embodiment is described below referring to FIGS. 5, 20 and 21.

When the film 330 is peeled away from the state shown in FIG. 20 to remove the holder 800 and the positioning tape 300 from inside the film 330, the peel-off paper 810 is moved together with the film 330 an peeled from the device-side adhesion surface 311 (321) of the positioning tape 300. As a result, the positioning tape 300 remains held by the holder 800 and the sticky device-side adhesion surface 311 (321) is exposed. As shown in FIG. 21, after the holder 800 and the positioning tape 300 have been removed from the film 330, the holder 800 still retaining the positioning tape 300 is fitted into the skin guide 120 from the bottom side (bottom 122 side) installed in the piercing device 100. At this time the device-side adhesion surface 311 (321) of the positioning tape 300 retained in the holder 800 is adhered to the bottom 122 of the skin guide 120. Since the holder 800 is positioned relative to the skin guide 120, the positioning tape 300 is adhered at a predetermined position on the bottom 122 of the skin guide 120. Specifically, the pair of two-sided tape pieces 310 and 320 of the positioning tape 300 are adhered to the bottom 122 of the skin guide 120 at a mutual spacing of distance D1 (approximately 30 mm) so that the processing region (region in which micropores are formed) 510 of the piercing device 100 is approximately centered in the circumscribed region, as shown in FIG. 5.

Note that in other aspects the structure of the third embodiment is identical to that of the first embodiment.

In the third embodiment described above, a single skin guide 120 can be reused repeatedly by packaging the unused positioning tape 300 in the film 330 with the positioning tape 300 separated from the skin guide 120.

Note that in other aspects the effect of the third embodiment is identical to that of the first embodiment.

Note that the embodiments of the present disclosure should be regarded as examples in all aspects and in no way limiting. The scope of the present invention is defined by the scope of the claims and not be the description of the embodiment, and includes all modifications within the scope of the claims and the meanings and equivalences therein.

For example, although the first through third embodiments have been described by way of the example of a measuring apparatus for measuring glucose level and electrolyte concentration, the present invention is not limited to this arrangement in as much as measuring devices other than a measuring apparatus for measuring glucose level and electrolyte concentration may be used if the measuring apparatus measures the components in a living body contained in tissue fluid. The glucose level also may be measured indirectly by substituting another value for the glucose level.

Although the first and third embodiments are described by way of example as structures providing triangular notches on the positioning tape as a marking member and triangular projections in the holding part of the collecting member so that the collecting part of the collecting member is adhered to the processing region by matching the positioning the notches and projections, the present invention is not limited to this arrangement in as much as a mark 901 (for example, a triangular mark) may be inscribed beforehand on the surface of the positioning tape 300 as a marking member and a mark 911 (for example, a triangular mark) may be inscribed beforehand beforehand on the surface of the holding part 910 so that the collecting part 400a may be adhered to the processing region 510 by matching the positions of the mark 901 and the mark 911, as shown in FIG. 22.

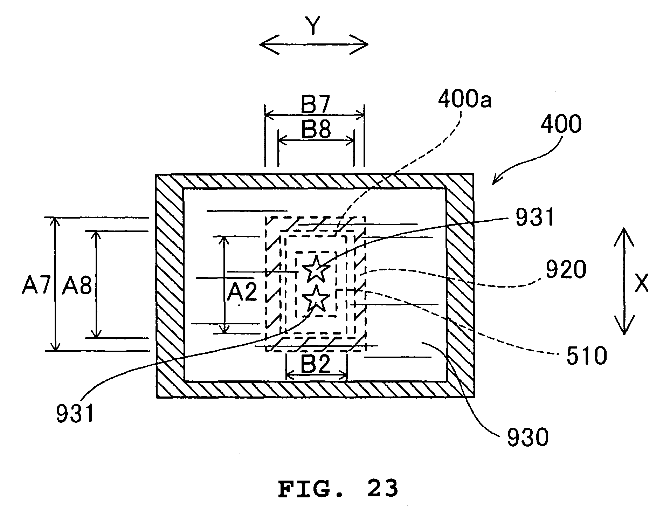

Although the first through third embodiments are described by way of example of structures in which the marking member positioning tape is peeled from the skin 500 after the collecting member holding part has been adhered to the skin, the present invention is not limited to this arrangement in as much as the holding part 930 may also be adhered to the skin so as to cover the marking member positioning tape, As shown in FIG. 23.

Specifically, the external shape of the positioning tape 920 is approximately rectangular with a length A7 in the X direction of approximately 16 mm and a length B7 in the Y direction of approximately 11 mm. An approximately rectangular empty space with a length A8 in the X direction of approximately 14 mm and a length B8 in the Y direction of approximately 9 mm is formed in the positioning tape 920. That is, the positioning tape 920 is frame-like in shape and the frame is approximately 1 mm in width. The positioning tape 920 is arranged so that the processing region (region in which micropores are formed) 510 of the piercing device 100 is disposed in the approximate center of the region circumscribed by the frame-like positioning tape 920. The collecting part 400a of the collecting member 400 is formed with an exterior shape (length A2 in the X direction of approximately 12 mm and length B2 in the Y direction of approximately 7 mm) is slightly smaller than the center part (length A8 in the X direction of approximately 14 mm and length B8 in the Y direction of approximately 9 mm). A mark 931 (for example, a star-shaped symbol) is inscribed beforehand at a position corresponding to the collecting part 400a on the surface of the holding part 930 of the collecting member 400.

The holding part 930 is adhered to the skin so that the mark 931 is disposed within the empty space of the positioning tape 920 adhered to the skin, such that the processing region (region in which micropores are formed) 510 is thereby circumscribed. Thus, the collecting part 400a disposed at the position corresponding to the mark 931 is accurately adhered to the processing region (region in which micropores are formed) 510.

In this case, the positioning tape 920 can not be peeled from the skin since the positioning tape 920 is covered by the holding part 930. However, the positioning tape 920 is formed in an extremely small configuration so as to be of a minimum size required to arrange the collecting part 400a within the empty space in a planar view as shown in FIG. 23. A positioning tape of such a shape is small and reduced the burden on the user (patient).

The entirety of the positioning tape need not be covered by the holding part in as much as the holding part that holds the collecting part also may be adhered to the skin so as to overlap part of the positioning tape. In this case, if a mark is inscribed beforehand on the positioning tape and a mark is inscribed beforehand on the holding part, the holding part can be positioned using the respective marks.

Although the first through third embodiments have been described by way of examples in which a marking member positioning tape is adhered to the skin to indicate the position of the processing region for the processing (micropore formation) performed by the piercing device, the present invention is not limited to this arrangement in as much as the processing may be performed by a micropore forming device in a region indicated by a marking member when the marking member has been adhered to the skin beforehand.

Although the first through third embodiments describe a holding part formed of film (thin film) as an example of a holding part, the present invention is not limited to this arrangement in as much as a three dimensional solid shape holding part having a thickness greater than a film holding part is also possible. In this case the three-dimensional solid shape holding part does not require a reinforcing member since the mechanical strength is greater than that of the film.

Although the first through third embodiments describe by way of examples structures in which the adhesive force between the positioning tape and the skin guide is smaller than the adhesive force between the positioning tape and the skin by providing a silicon coating on the bottom of the skin guide, the present invention is not limited to this arrangement in as much as the adhesive force between the positioning tape and the skin guide may be smaller than the adhesive force between the positioning tape and the skin by making the adhesion area between the positioning tape and the skin guide so as to be less than the adhesion area between the positioning tape and the skin. The adhesive force between the positioning tape and the skin guide may also be made less than the adhesive force between the positioning tape and the skin by reducing the amount of adhesive on the adhesion surface between the positioning tape and the skin guide so as to be less than the amount of adhesive on the adhesion surface between the positioning tape and the skin.

Although the first through third embodiments have been described by way of examples in which the collecting part is adhered to the processing region by positioning the holding part relative to the marking member positioning tape, the present invention is not limited to this arrangement in as much as the collecting part also may be adhered to the processing region by positioning the collecting part directly relative to the positioning tape.

Although the first through third embodiments have been described by way of examples in which the positioning tape is configured as a marking member to indicate the position of the processing region by circumscribing the entirety of the processing region, the present invention is not limited to this arrangement in as much as a marking member may be configured to indicate the position of the processing region by circumscribing only a part of the processing region, and the marking member also may be configured to indicate the position of the processing region without circumscribing the processing region. In this case, for example, the marking member may be configured so that the leading end of the marking member indicates the position of the processing region by forming the marking member in an arrow shape.

Although the first through third embodiments have been described by way of examples in which a holding part is provided to hold the collecting part on the collecting member, the present invention is not limited to this arrangement in as much as the collecting part may be adhered to the processing region by providing the collecting part with an adhesion surface capable of adhering to the skin and adhering this adhesion surface to the skin.

## Claims

1. A system for measuring components in a living body comprising:
a micropore forming device for forming micropores by moving a microneedle chip toward a skin;
a guide member comprising:
a mounting part being mounted on one end of the micropore forming device; and
a positioning part for positioning the micropore forming device on the skin,
wherein the guide member is provided with a channel permits a passage of the microneedle chip when the microneedle chip is moved toward the skin;
a marking member, for indicating a position on the skin on which micropores are formed, comprising:
a first adhesive surface adhered to the positioning part; and
a second adhesive surface, on the back surface of the first adhesive surface, adherable to the skin by a greater adhesion force than the adhesion force of the first adhesive surface to the positioning part;
a collecting member, for collecting tissue fluid from the skin, placed at a position indicated by the marking member on the skin; and
a measuring apparatus for measuring components in a living body contained in the tissue fluid collected by the collecting member.

2. The system for measuring components in a living body of claim 1, wherein
the marking member comprises:
a first marking member provided on the positioning part; and
a second marking member provided at a position, opposite the first marking member with the opening of the channel, on the positioning part,
wherein the first marking member and the second marking member respectively comprise the first adhesive surface and the second adhesive surface.

3. The system for measuring components in a living body of claim 1 or 2, wherein
the marking member has a configuration capable of surrounding the opening of the channel of the guide member.

4. The system for measuring components in a living body of any one of claims 1 to 3, wherein
the collecting member comprises:
a collecting part for collecting tissue fluid; and
a holding part, for holding the collecting part, that is adherable to the skin,
wherein the marking member has a predetermined configuration, and
the holding part has a configuration correspond to the configuration of the marking member.

5. The system for measuring components in a living body of claim 4, wherein
the collecting part is a gel; and
the holding part is a film.

6. The system for measuring components in a living body of claim 4 or 5, wherein
the holding part is water-proof, and is large enough to cover the collecting part placed on the skin.

7. A kit for a micropore forming device for use in a micropore forming device for forming micropores by moving a microneedle chip toward a skin, comprising:
a guide member comprising:
a mounting part being mounted on one end of the micropore forming device; and
a positioning part for positioning the micropore forming device on the skin,
wherein the guide member is provided with a channel permits a passage of the microneedle chip when the microneedle chip is moved toward the skin;
a marking member, for indicating a position on the skin on which micropores are formed, comprising:
a first adhesive surface adhered to the positioning part; and
a second adhesive surface, on the back surface of the first adhesive surface, adherable to the skin by a greater adhesion force than the adhesion force of the first adhesive surface to the positioning part.

8. The kit for a micropore forming device of claim 7,
wherein
the marking member comprises:
a first marking member provided on the positioning part; and
a second marking member provided at a position, opposite the first marking member with the opening of the channel, on the positioning part,
wherein the first marking member and the second marking member respectively comprise the first adhesive surface and the second adhesive surface.

9. The kit for a micropore forming device of claim 7 or 8,
wherein
the marking member has a configuration capable of surrounding the opening of the channel of guide member.

10. The kit for a micropore forming device of any one of claims 7 to 9, further comprising:
a collecting member for collecting tissue fluid from the skin by being placed at a position on the skin on which micropores have been formed.

11. The kit for a micropore forming device of claim 10,
wherein
the collecting member comprises:
a collecting part for collecting tissue fluid; and
a holding part, for holding the collecting part, that is adherable to the skin,
wherein the marking member has a predetermined configuration, and
the holding part has a configuration corresponding to the configuration of the marking member.

12. The kit for a micropore forming device of claim 11,
wherein
the collecting part is a gel; and
the holding part is a film.

13. The kit for a micropore forming device of claim 11 or 12, wherein
the holding part is water-proof, and is large enough to cover the collecting part placed on the skin.

14. The kit for a micropore forming device of any one of claims 7 to 13, wherein
the marking member is configured as a sheet.

15. A marking member, for indicating a position on a skin on which micropores have been formed, configured to adhere to a guide member, wherein the guide member comprises a mounting part being mounted on one end of the micropore forming device for forming micropores by moving a microneedle chip toward the skin and a positioning part for positioning the micropore forming device on the skin,
wherein the guide member is provided with a channel permits a passage of the microneedle chip when the microneedle chip is moved toward the skin, the marking member comprising:
a first adhesive surface adhered on the positioning part of the guide member; and
a second adhesive surface, on the back surface of the first adhesive surface, adherable to the skin,
wherein the marking member has a configuration capable of surrounding the opening of the channel.
